# EUROPEAN PATENT APPLICATION

(11) **EP 2 161 039 A1**
(43) Date of publication of application: **10.03.2010**
(21) Application number: 09010865.5
(22) Date of filing: 25.08.2009
(51) Int. Cl.: A61K 49/00, G01N 33/50, G09B 23/00

(54) **Method for testing an agent for strokes in humans with a non-human stroke model**

(30) Priority: 25.08.2008 US 91661 P; 19.08.2009 US 543528
(71) Applicant: THE OHIO STATE UNIVERSITY RESEARCH FOUNDATION, Columbus OH 43212-1154 (US)
(72) Inventor: Sen, Chandan K., New Albany, Ohio 43054 (US); Rink, Cameron, Galloway, Ohio 43119 (US); Roy, Sashwati, New Albany, Ohio 43054 (US); Christoforidis, Greg, Columbus, Ohio 43220 (US)
(74) Representative: Jennings, Tara Romaine

(57) **Abstract**

An agent for treating strokes in humans is tested in a non-human subject by selecting an agent for testing and by preparing a selected non-human subject. The preparation includes: inducing a stroke event by advancing a microwire through the arterial system of the subject to a selected intracranial target position, inserting a microcatheter along the microwire and delivering an embolic device to the target position, occluding the artery at the target position by deploying the embolic device, verifying the occlusion and repositioning the embolic device if needed. After a predetermined occlusion interval, reperfusion of the subject is simulated by removing the embolic device and commencing therapy with the selected agent. At appropriate intervals, the effect of the conducted course of therapy is assessed non-invasively until terminal evaluation. In particular aspects, the method involves occluding the middle cerebral artery through an access achieved via the basilar artery.

## Description

### Technical Field

Exemplary embodiments are directed to methods for testing, in a non-human mammal, potential agents for therapeutic treatment of stroke in a human. More particularly, the methods disclosed herein relate to inducing a controlled experimental stroke event in a large mammal, most particularly, a canine, to prepare the mammal for use as a stroke model.

### Background of the Art

Stroke is currently the leading cause of serious long-term disability and the third leading cause of death in the United States, with 780,000 Americans afflicted by a new or recurring stroke each year, as reported by Rosamond, et al [1]. Although a variety of therapeutic approaches have shown promise in small-animal models of stroke, the vast majority of clinical trials to test the efficacy of such modalities have failed, as reported by Lodder [2] and Van Reempts [3]. As of 2000, 75 different therapeutic strategies have been tested in acute stroke clinical trials, but only two have been widely accepted of proven benefit- aspirin and tissue plasminogen activator ("TPA"),as reported by Kidwell, et al [4]. The inadequacy of previously reported stroke models has been cited as a contributor to the lack of success for potential stroke therapeutics in clinical trials in the 2008 request for applications from the National Institutes of Health and Canadian Stroke Network for a Stroke Preclinical Trials Consortia in recognition of "the translational barriers that exist today in stroke research" [5].

Accordingly, there is an unmet need to provide a robust and reproducible pre-clinical stroke trial protocol, based on a non-human mammal, that can serve as an ideal proving ground for potential therapeutic agents.

### Summary

This and other unmet advantages are provided by the system and method described and shown in more detail below. According to an aspect of the present invention there is provided a method for testing, in a non-human mammal, an agent for treating stroke in a human, as specified in any one of claims 1-8, and a method for inducing an experimental stroke event in a non-human mammal, as specified in any one of claims 9-15.

An exemplary embodiment includes a large-animal, pre-clinical stroke model system and method to bridge the translational gap between laboratory and clinical research. Using an endovascular approach, an embolic device is intravascularly guided through the vertebrobasilar system under fluoroscopy to occlude the desired intracranial vessel. Following a period of occlusion, the embolic device is retrieved to simulate reperfusion. High-resolution magnetic resonance imaging may be employed to characterize the stroke lesion. Benefits of exemplary embodiments of this pre-clinical model include a minimally invasive approach, high-reproducibility, and the modeling of stroke pathology in a large animal system that closely approximates that of humans.

### Brief Description of the Drawings

A better understanding of the disclosed embodiments will be obtained from a reading of the following detailed description and the accompanying drawings, wherein identical reference characters refer to identical parts and in which:
**FIGURE 1** schematically depicts a portion of the arterial system of a non-human mammal, specifically, a canine;
**FIGURE 2** is a fluoroscopic image of the canine vertebrobasilar system;
**FIGURE 3** depicts c-arm fluoroscopy enabled real-time verification of MCA occlusion and reperfusion in a canine brain;
**FIGURE 4** depicts MRI imaging of coronal slices of MCA territory stroke-induced brain lesion;
**FIGURE 5****.**depicts volumetric reconstruction of the stroke-induced infarct lesion; and
**FIGURE 6** depicts histological analysis of post-stroke infarct and control tissue.

### Detailed Description

Unless otherwise defined, all technical and scientific terms used herein have the same meaning as commonly understood by one of ordinary skill in the art to which this invention pertains. Although methods and materials similar or equivalent to those described herein can be used in the practice or testing of the exemplary embodiments; suitable methods and materials are described below. All publications, patent applications, patents, and other references mentioned herein are incorporated by reference in their entirety. In case of conflict, the present specification, including definitions, will control. In addition, the materials, methods, and examples are illustrative only and not intended to be limiting.

The goal of the current work is to address a gap between laboratory and clinical research, by providing a novel pre-clinical model of acute focal ischemia in canines using an interventional radiology approach. The model developed is directed at canines, due to numerous advantages, anatomical and otherwise. The developed model, however, may be adapted to a number of large animals. The size and anatomical feature set of the canine brain mimics human brain more closely than small animal stroke models. Canines have a highly evolved gyrencephalic neocortex with a white to gray matter ratio that more closely approximates humans, as noted in references [6] and [7]. The canine neurovascular architecture accommodates an array of endovascular devices and interventional radiology techniques permitting a minimally invasive approach to the surgery while providing real-time visualization of occlusion.

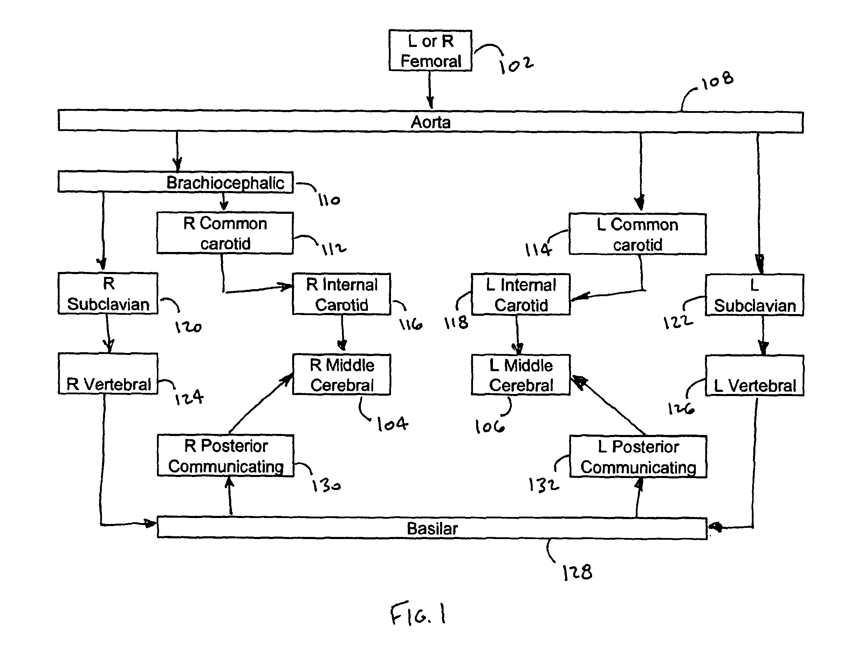

Referring now to **FIGURE 1****,** a schematic diagram shows arterial pathways in a non-human mammal leading from one of the femoral arteries **102,** a conventional entry point for catheterization, to one of the middle cerebral arteries (MCA) **104, 106,** which is an intercranial occlusion target. Since approximately 75% of all human acute ischemic stroke case affect the MCA, this target is of particular relevance and interest. While this particular pathway is described in detail, it is to be understood that this pathway is not the sole pathway that is useful in practicing the methods disclosed herein. As in a human, a model for intraluminally occluding an intracranial artery starts with access to the cerebrovascular system gained by advancing a microwire from the selected right or left femoral artery **102** into the aorta **108** and past the arch of the aorta (not shown). At that point, the microwire may be passed into one of the common carotids. Access is made by way of the brachiocephalic artery **110** to the right common carotid **112,** although access to the left common carotid artery **114** is made directly from the aorta **108.** From the selected common carotid artery **112, 114,** the microwire may be advanced into and through the corresponding internal carotid artery (ICA) **116, 118** by avoiding entry into the corresponding external carotid artery (not shown). Once into the selected internal carotid artery **116, 118,** access is made to the corresponding middle cerebral artery **104, 106.** In the canine animal, this approach through the internal carotid artery **116, 118** is not feasible using microcatheter techniques, as the ICA is tortuous. Navigation with microwires as small as 0.010" in diameter is effectively prevented.

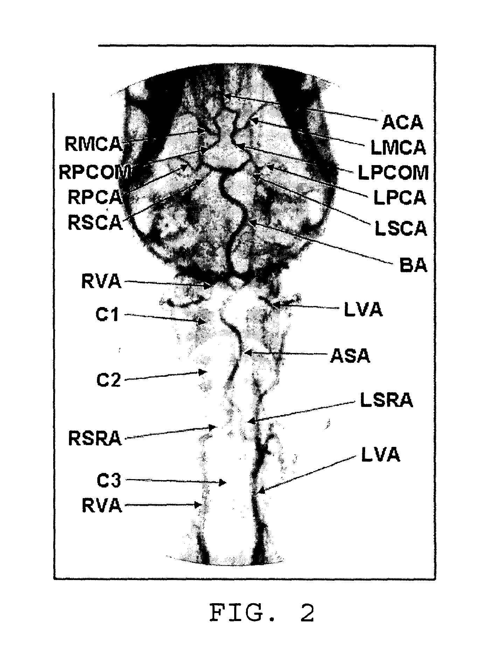

Still with reference to Fig. 1, and as an alternative approach, the microwire can be advanced from a selected right or left femoral artery **102** into the aorta **108.** If the microwire is directed to the right side, the right subclavian artery **120** is reached by passing through the brachiocephalic artery **110** to a point past the right common carotid **112.** On the left side, the left subclavian **122** is located past the left common carotid **114.** Either subclavian artery **120, 122** provides access, through the corresponding right and left vertebral artery **124, 126**, to the basilar artery (BA) **128.** From here, and along a selected one of the right and left posterior communicating arteries **130, 132,** the corresponding right or left middle cerebral artery **104, 106** is reached. After this, a microcatheter is positioned into the selected MCA. A 3 mm x 20 cm Ultrasoft Matrix2 coil, such as that available from Boston Scientific, Natick, MA, is deployed for 1 hour, providing a controlled occlusion of the MCA. After the 1 hour of occlusion, the coil is retrieved. The MCA territory is reperfused as confirmed under fluoroscopy. In addition to landmarks already discussed, the fluoroscopic view of **FIGURE 2** shows: the left and right vertebral arteries LVA, RVA; vertebrae 1 through 3 C1, C2, C3; the left and right spinal ramus arteries LSRA, RSRA; the anterior spinal artery ASA; the left and right superior cerebellar arteries LSCA, RSCA; the left and right posterior cerebral arteries LPCA, RPCA; and the anterior cerebral artery ACA.

Beyond the specific target location and pathway, alternative occlusion sites would include the internal carotid arteries 116, 118, the anterior cerebral artery, the posterior cerebral artery, the posterior communicating arteries **130, 132,** the superior cerebellar arteries and the basilar artery **128.**

Although a specific coil is cited above, the endovascular mid-cerebral artery (MCA) occlusion can also be achieved with a coil comprising platinum/iridium, platinum tungsten, or platinum/nickel. A soft platinum matrix coil that has been clinically used (as reported in reference [8]) to treat intracranial aneurisms. This approach necessitates only two small femoral artery punctures to navigate the neurovasculature as described in Fig 1 and deploy the device in the M1 segment of the MCA under guided fluoroscopy.

Because the described MCA occlusion in canines offers a highly reproducible and relatively inexpensive alternative to non-human primate models of acute focal ischemia, it is preferred thereto. Although a non-human primate is anatomically closer to a human than a canine, the use of a non-human primate poses additional ethical, veterinary and housing considerations that obligate larger fiscal and personnel requirements [9], [10].

### EXAMPLE 1

### Materials and Methods

*Endovascular Canine MCA Occlusion.* All experimentation was approved by the Institutional Laboratory Animal Care and Use Committee of The Ohio State University. One day prior to percutaneous intervention, mongrel canines (n=4) with a body weight of 20-30kg received a 300 mg loading dose of clopidogrel. On the day of surgery, the animals were sedated with telazol (6 mg/kg bw intramuscular, volume <3cc) and anesthetized (1.5-2.0% isoflurane). Continuous cardiac rhythm, respiration rate, end-tidal CO2, and oxygen saturation were monitored for control of physiologic parameters. Canine body temperature was maintained around the normal range of 38-39.2°C using a convective warming system (Gaymar Thermacare, Orchard Park, NY). Access to the bilateral common femoral artery was obtained using 5 French sheaths (Arrow, Erding, Germany). Under fluoroscopic guidance (GE Medical OEC 9800 Plus Cardiac, GE Healthsystems, Piscataway, NJ), a five French guide catheter (Boston Scientific, Natick, MA) was advanced into the vertebral artery (VA) and was hooked up to a pressurized drip. The animal was administered 2000 units of heparin as a bolus. A 4 French catheter (Boston Scientific) was then placed in the right vertebral artery to provide access into the basilar artery system and allow for periodic infusion of spasmolytic agents as needed (papaverine 0.3mg/ml delivered at 1 ml aliquots). Arteriography allowed for documentation of vasodilatation in the intended territory. The MCA was accessed through microcatheter techniques by way of the circle of Willis, as seen in Fig. 1. Through the vertebral artery (VA) guide catheter a SL-10 microcatheter (Boston Scientific) with a microwire was advanced into the MCA. Once the microcatheter was in place, an embolic coil (3x20 Ultrasoft Matrix2 Platinum Coil, Boston Scientific) was delivered into either MCA to occlude the entire M1 segment. The 4F catheter and the 5F catheter were then used to perform digital subtraction angiograms (DSAs) of the internal carotid arteries and the vertebrobasilar circulation, confirming complete occlusion of the MCA via fluoroscopic contrast injection without evidence for either circle of Willis collaterals or any pial collateral formation that may have developed during the occlusion. If needed, the coil was repositioned to achieve complete occlusion. Angiographic evidence for incomplete occlusion or pial collateral formation reconstituting the occluded territory were considered exclusion criteria for MRI analysis.

Once the coil was properly positioned, the microcatheter was drawn back into the third spinal arterial ramus. DSAs of the ICA and VA were repeated every 15 minutes to confirm continued occlusion. Occlusion of the MCA lasted for 1 hour. At the end of the transient occlusion, the microcatheter was advanced again into the MCA and the coil was captured and retrieved, followed by DSAs of the VA and ICA to confirm reperfusion of the occluded territory. The catheters were then removed. A blood draw for activated clotting time was then obtained. Depending on the result of the activation time, a weight based calculated dose of protamine was delivered to the canine to reverse the effects of heparinization. The sheaths were then removed and pressure was applied at the arteriotomy sites for hemostasis. The canine was then brought out of anesthesia, extubated and the arteriotomy sites were periodically checked for hematoma. Post-operative veterinary care was provided to the canines for 24 hours prior to MRI.

*Magnetic Resonance Imaging (MRI).* The infarct lesion was evaluated using 3T MRl (Philips Healthcare, Andover, MA) 24 hours after MCA reperfusion. The animal was sedated with telazol (6 mg/kg body weight, intramuscular, volume <3cc) and anesthetized (1.5-2.0% isoflurane) throughout the MRI scans (approximately 1 hour). While under anesthesia in the magnet, the heart rate, respiratory rate and body temperature were monitored. All MR imaging was performed under the guidance and supervision of a trained technician at the Wright Center of Innovation for Biomedical Imaging (Columbus, OH). The image processing software ImageJ (NIH, Bethesda, MD) was used for infarct volume calculation from coronal T2-weighted MR images (3mm slice thickness). Raw MR images were converted to standard DICOM (Digital Imaging and Communications in Medicine) format and transferred to an image processing workstation. After appropriate software contrast enhancement of the images, the infarct region, ipsilateral hemisphere and contralateral hemisphere were delineated by manual planimetry performed by two independent observers. This technique was used to quantify stroke injury as a fraction of contralateral hemisphere and total brain volume. Correction for edema induced midline shift in hemispherical volume was incorporated into infarct volume calculations as described previously in reference [12]. Interobserver reproducibility was assessed using the Bland-Altman statistic described in reference [37].

*Histology.* Immediately following the MRI, the subject canines were euthanized (euthasol, IV, 1 ml/4.6kg) and the canine brains were isolated by necropsy. Continuous 3mm coronal slices were collected throughout the ipsilateral and contralateral hemispheres using a canine brain matrix. Sections were rinsed in PBS, embedded in OCT compound (Sakura Finetek, Torrance, CA) and frozen at -80°C. These OCT-embedded slices were subsequently cut in 10µm thick sections on a Leica CM 3050 S cryostat (Leica Microsystems, Wetzlar, Germany) and mounted onto slides for histological determinations. Hematoxylin and eosin staining of frozen canine brain tissue was performed to contrast gross stroke pathology in infarct affected and contralateral control tissue.

Fluoro-Jade is an anionic fluorochrome capable of selectively staining degenerating neurons in brain slices. Compared to conventional methodologies, Fluoro-Jade is a sensitive and more definitive marker of neuronal degeneration than Nissl-type stains, while also being comparably sensitive yet considerably simpler and more reliable than suppressed silver techniques [38]. To determine neuronal degeneration, the frozen brain sections (10µm) were stained using a known Fluoro-Jade procedure, such as that provided in reference_[39]. Tissue sections were analyzed by fluorescence microscopy (Axiovert 200M) and images were captured using Axiovert v4.6 software (Zeiss, Germany).

### EXAMPLE 2

### Results

The intuitive endovascular access to the MCA is by way of the internal carotid artery (ICA). As a result, there is a deficiency of published MCA occlusion models that purposefully explore alternative routes, as noted in reference [11]. In the canine animal, the ICA to MCA approach was determined to be not feasible, due to tortuosity of the canine ICA. However, the basilar artery (BA) to MCA approach was determined to be effective to provide the required endovascular access. When navigation of the canine ICA was attempted with an array of small diameter microwire (0.010" - 0.014") and microcatheter systems, the probes were not capable of advancing beyond the cavernous portion of the ICA using standard microcatheter techniques. However, and as seen in Fig 1, endovascular approach through the basilar artery was possible. The basilar artery is adequately large and straight to accommodate the FASdasher 14 microwire and SL-10 microcatheter. Insertion of the catheter into the femoral artery (FA) allowed access to the vertebral arteries (VA) that branch off of the left and right subclavian arteries near the aortic arch. The microwire was advanced from either VA into the anterior spinal artery (ASA) via the spinal ramus artery (SRA). Intracranially, the ASA continues to become the BA. By directing the microwire from the BA around the Circle of Willis, entry was made into either the left or right MCA, the selection depending on which side appeared more favorable to access under C-arm fluoroscopy. The potential of vasospasm of the posterior communicating (PCOM) artery and BA necessitated constant monitoring of contrast filling under fluoroscopy to assure that neither is unintentionally occluded by the microwire or microcatheter and Inadvertent BA and PCOM occlusion was documented in preliminary studies.

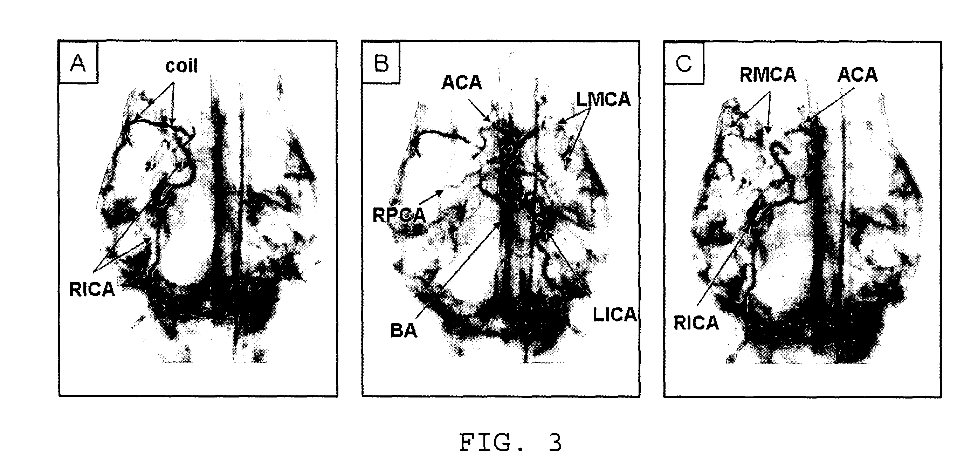

To reduce the risk of inadvertent occlusion, the matrix coil was quickly deployed into the MCA upon successful tracking of the microcatheter across the PCOM, as shown in **FIGURE 3A****.** After placement of the matrix coil in the M1 segment of the MCA, the microcatheter was then retreated to the origin of the BA. Successful MCA occlusion was verified by ICA injection of contrast agent under fluoroscopy. During the MCA occlusion, both ICAs were routinely subjected to arteriograms to confirm occlusion of the ipsilateral MCA and opacification of the contralateral hemisphere as seen in **FIGURE 3B****.** After 1 hour of occlusion, the matrix coil was retrieved and patency of the MCA territory confirmed reperfusion as seen in **FIGURE 3C****.** Slower than normal filling of the reperfused MCA was documented and presumed to be a consequence of vasospasm. A post-reperfusion BA arteriogram was performed in order to confirm opacification of the PCOMs and both MCAs. Physiological parameters, including heart rate, respiration rate, oxygen saturation ("SaO₂") and end-tidal CO2 ("ETCO₂") were monitored and maintained stable prior to, during and following MCA occlusion. A table of physiological parameters from this testing is as follows:

| Time | -30 min | 0 min | 30 min | 60 min | 90 min |
|---|---|---|---|---|---|
| Heart rate (min⁻¹) | 113.4 ± 11.3 | 114.0 ± 7.4 | 116.6 ± 7.6 | 115.6 ± 10.5 | 113.6 ± 12.3 |
| Respiration (min⁻¹) | 14.2 ± 7.8 | 14.4 ± 6.3 | 13.4 ± 4.2 | 15.0 ± 6.1 | 13.0 ± 2.7 |
| SaO₂ (%) | 94.6 ± 2.9 | 94.8 ± 3.4 | 96.8 ± 1.6 | 97.4 ± 1.3 | 96.2 ± 1.6 |
| ETCO₂ (mmHg) | 17.5 ± 6.9 | 16.8 ± 4.3 | 17.3 ± 3.3 | 17.0 ± 3.4 | 17.3 ± 3.3 |

In the above Table, the -30 minute datum is a reading taken pre-MCA occlusion, the 90 minute datum is a post-occlusion reading and the three intermediate data points represent readings taken at onset, middle and end of the 1 hour occlusion. The values shown above are mean values ± standard deviation.

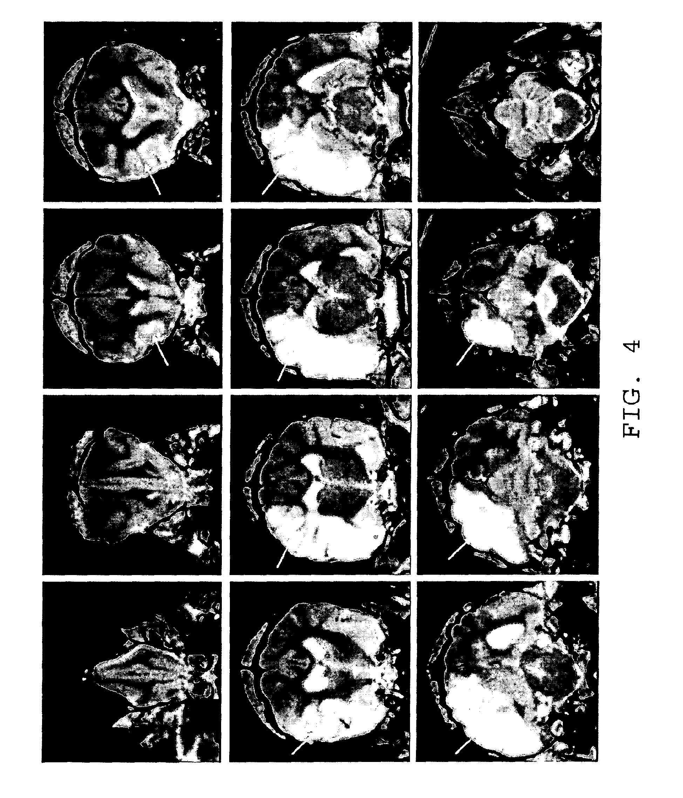

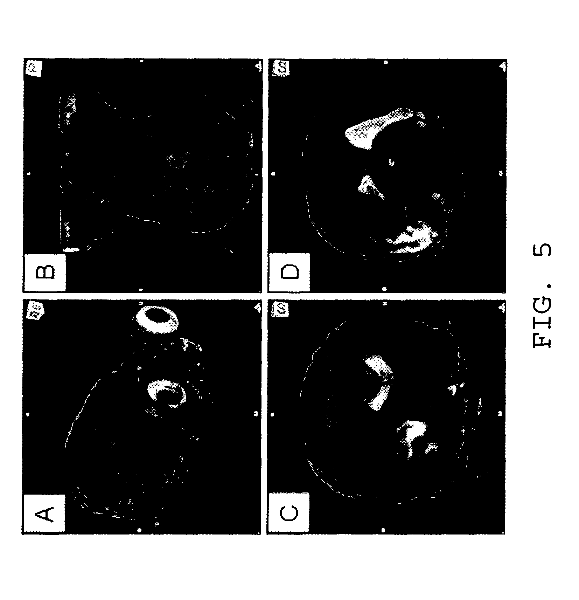

Twenty-four hours after MCA reperfusion, high resolution T2-weighted images of coronal brain slices from 3T MRI revealed infarct associated with the region of the cerebral cortex supplied by the MCA, as shown in **FIGURES 4** and **5****.** After the matrix coil is deployed into the RMCA, occlusion of the M1 segment as was confirmed by RICA contrast injection, as seen in Fig. 3A. Figure 3B shows LICA angiography demonstrating that the LMCA is still intact, along with the ACA, BA, and RPCA. The RMCA still does not fill. Figure 3C illustrates that; after 1 hr of occlusion and retrieval of the matrix coil, the RMCA has reperfused, as seen through RlCA arteriogram.

**FIGURE 4** depicts MRI imaging of Coronal slices of MCA territory stroke-induced brain lesion. T2-weighted MRI (TR=3000ms, TE=100ms, Field of View=145mm, Slice Thickness=3.0mm, Echo Train Length=15, Acquisition Matrix=256x256, Number of Averages=2) was performed 24 hr after reperfusion of the MCA using a Phillips 3T system. Coronal slices demonstrating cortical edema (arrow) are ordered from anterior (top left) to posterior (bottom right) cortex;

Manual planimetry was used to quantitate infarct volume by delineating the infarct region, ipsilateral and contralateral hemispheres. Hemispheric volumes were determined from T2-weighted images by use of the following neuroanatomic landmarks: falx cerebri, pineal gland, fissure longitudinalis, infundibulum, sylvian aqueduct, and third ventricle. Lesion areas were then summed and multiplied by the slice gap thickness to obtain an infarct volume uncorrected for edema. A substantial midline shift, evidenced by the displacement of the third ventricle, is characteristic of the supratentorial lesion. As a result, the relative hemispherical size is distorted which in turn leads to a misrepresentation of true hemispherical infarct volume. Since there was a strong presence of edema induced swelling in canine brain at 24 hour post MCA reperfusion, a method for edema corrected lesion volume calculation, as reported in reference [12], was used. This calculation is based on three assumptions: 1) compression of the contralateral hemisphere is comparable to compression of the entire healthy brain tissue, whereas the lesion is not compressed; 2) the contralateral hemisphere is compressed to the same extent as the affected hemisphere is extended, but total brain volume does not change; and 3) volume extension occurs only within the lesion, not in the unaffected tissue. Taking these factors into account, the mean percent hemispherical infarct volume corrected for edema in the four canines as reviewed by two independent observers was 30.9± 2.1 and 31.2± 4.3. Applying Bland-Altman observer comparison of ratio (observer 1/observer 2) vs. average, the bias.= 1.0003±0.066.

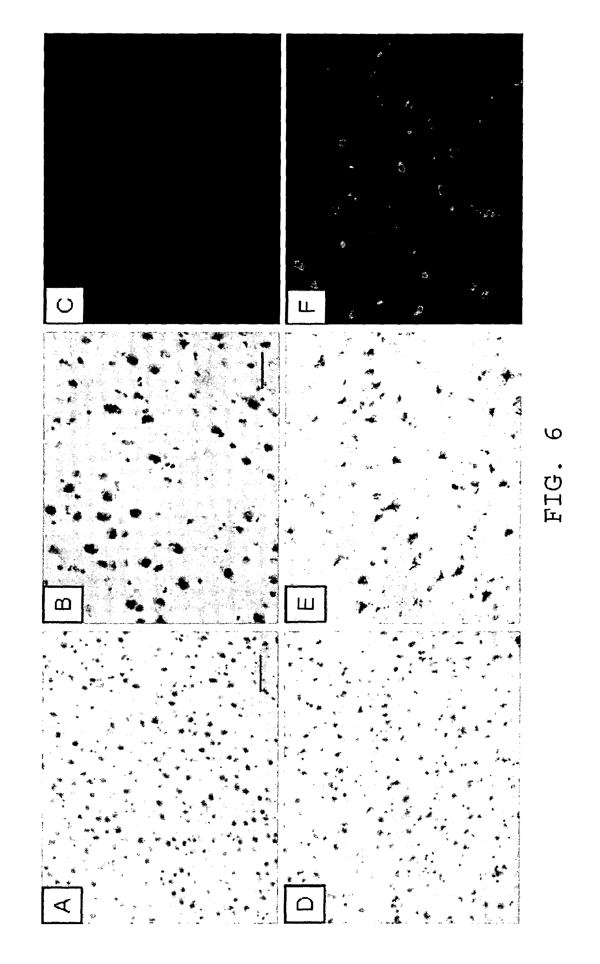

Three dimensional reconstruction of axial MRI slices using Osirix software provided a clear visual representation of infarct size in relation to hemispherical volume. Hematoxylin and eosin staining of the stroke-induced infarct tissue of the neocortex at 24 hr time point revealed condensed, pyknotic nuclei as compared to contralateral control tissue, as seen in **FIGURES 6A****, B, D, E.** Positive Fluoro-Jade immunofluoresence staining indicated neuronal degeneration in infarct affected tissue as compared to contralateral controls, as seen in **Figs 6C****, F.** In these volumetric reconstructions of stroke-induced infarct lesion, T2-weighted MRI coronal slices (acquisition details in Fig. 3) were volume rendered. Color contrast from black to white represents increasing signal intensity. The eyes of the test subject have been kept in the field of view for orientation purposes (Figs 5A and 5B). Stroke-induced infarct appears as light color throughout a large portion of the right hemisphere in Fig 5A, which is an oblique view and Fig 5B, which is anterior/posterior view. Coronal sectioning through the brain reveals MCA territory infarct and ventricular compression in the right hemisphere (Figs 5C and 5D in oblique view).

**FIGURE 6** depicts histological analysis of post-stroke infarct and control tissue. Following 24 hr MRI, canines (n=4) were euthanized and brain tissue was cryosectioned for histological analyses. H&E staining of non-infarcted contralateral hemisphere tissue is shown in Fig 6A and infracted stroke tissue, in Fig 6D, at 10x magnification. H&E staining of non-infarcted contralateral hemisphere tissue is shown in Fig 6B and infracted stroke tissue, in Fig 6E at 20x magnification. Nucleus (blue, DAPI) and degenerative neuron staining (green, FluoroJade) of non-infarcted contralateral hemisphere tissue is shown in Fig 6C and infarcted stroke tissue, in Fig 6F, at 20x magnification.

An intraluminal suture model of MCA occlusion in rats, which was first reported by Koizumi et al in 1986 [13] and later modified by Longa et al in 1989 [14], introduced a novel non-invasive small animal model of acute focal ischemia. This model continues to be the most frequently used method to test potential therapeutic stroke agents *in vivo* [15]. Several variations of techniques and approaches to induce MCA occlusion in rodents have been published since the model's inception, however, none have been as widely accepted, as noted in references [16] through [18]. In spite of the known and significant limitations to the intraluminal suture protocol, which have been extensively documented in the literature (as reported in references [2], [15] and [19] through [23]) and contribute to the high degree of variability in lesion volume and location across and within studies, the protocol is still used, which suggests lack of an alternative. The real-time placement of the occluder in the rodent MCA cannot be visualized. Consequently, overshooting or undershooting the MCA origin is expected to be a common occurrence that may contribute to variability in lesion volume outcomes. Laser Doppler flowmetry (LDF) clearly improves the reliability of occluder placement, but LDF only provides information on the relative decrease in blood flow at a single point, making it difficult to determine if the territory is fully or only partially occluded. Further contributing to the variability of stroke lesion volume in the intraluminal suture model is the potential for premature reperfusion of the MCA territory. This is documented to occur in 25% of experimental animals [15]. Finally, it has been reported in the intraluminal suture model that, after placement of the filament tip in the proximal MCA, the remaining filament in the ICA is occlusive to the anterior choroid artery and the hypothalamic artery, producing unintentional subcortical lesions [24].

The present method overcomes each of these limitations. The occlusion of the M1 section of the MCA may be appreciated in real-time using angiograms. This allows the entire ischemic territory to be visualized and confirms partial or full MCA occlusion. While premature reperfusion was not encountered in this study, repeated monitoring of the MCA territory via C-arm fluoroscopy allowed for adjustment of the matrix coil as needed to ensure complete occlusion of the MCA throughout the ischemic event. Based on the tests conducted, the occlusion has been seen to be specific to the MCA supplied territory. By retreating the microcatheter to the BA origin, unintentional occlusion of arteries, such as the PCOM, which remain patent under fluoroscopy, can be prevented.

The intraluminal suture model's high degree of variability in lesion volume is not limited to use in small animals such as rodents. In translating the intraluminal suture model to non-human primates, Freret et al [25] reported a 58% standard deviation around the mean infarct volume from marmosets (n=4) subjected to transient MCA occlusion. In contrast, the standard deviation around the mean (n=4) in canines using the current matrix coil approach was found to be less than 15% for both observers. Similarly, other endovascular models employing the ICA route to MCA occlusion in non-human primates have reported large standard deviations in infarct volume across animals [26], [27]. Because the standard deviation in stroke induced lesion volumes is appreciably tighter, it is expected that the present method would require fewer experimental animals to achieve statistical significance in studying potential therapeutic candidates. When compared to other large animal models of MCA occlusion, the present method offers a minimally invasive approach.

Several variations of transorbital MCA occlusion via vessel clamp in non-human primates have been reported, such as in references [28] through [32]. While variants of such models have reported highly reproducible infarct volumes, they necessitate an invasive approach including removal of the orbital globe, transection of the optic nerve and ophthalmic artery, and craniectomy of the posteromedial orbit [30]. To date, the effects of the orbital wound and trauma-induced inflammatory response have not been resolved separately from the stroke induced pathology. The craniectomy approach to model stroke in a pre-clinical setting is also associated with severe head trauma, changes in intracranial pressure, and cerebrospinal fluid loss [33], [34]. Because stroke is not necessarily associated with head trauma in humans, the trauma aspect of invasive surgery may be viewed as a major confounding factor. The close proximity of the trauma to the stroke site in these invasive models, including removal of the skull and dura covering the anterior circle of Willis, poses significant limitations. The minimally invasive nature of the present method represents a major strength. Of further consideration when working with non-human primates is the increased cost due to stringent enrichment program and housing guidelines [35], [36]. Although the standard of care remains the same, the relative cost of canine environmental enrichment and housing is considerably less as compared to non-human primates.

In performing the method described above, it is important to note that the canine vertebrobasilar system is prone to vasospasm that can arrest advance of the microcatheter and microwire. Significant precaution must be taken to avoid prolonged exposure of the microcatheter to the PCOM and the distal BA. Microwire movement must also be limited to the purposeful advancement of the wire to the MCA origin as quickly as possible to avoid spasm. If vasospasm occurs, waiting for it to resolve for a short interval (5-10min) or delivering a small dose of spasmolytic agent will generally overcome the problem. In some canines, variations of the cerebrovascular anatomy simply renders the described MCA cannulation too difficult. When this occurs, the catheters and wires should be removed, and the canine should be recovered and transferred out of the study.

Clearly, the goal of the invention is to provide a reproducible procedure, of a minimally-invasive nature, for providing test subjects for the testing of potential agents for therapeutic treatment of acute stroke in humans. Small animal models have been largely unsuccessful in this arena, as it has been difficult to have a tightly reproducible surgical procedures across and within studies. Also, there are vast anatomical and functional differences between small and large mammalian brains. The invasive transorbital approach, used in the past primarily with non-human primates, introduces trauma-associated complications that do not properly model many human strokes. Toward this end, the method of inducing a controlled stroke in a canine subject, followed immediately by the administration of a course of treatment with a selected potential therapeutic agent and followed subsequently by appropriate examination of the canine brain puts the medical researcher in possession of a powerful pre-clinical stroke model benefiting from guided fluoroscopic occlusion of the MCA, and high inter-animal reproducibility in a cost effective large animal setting.

### List of References

The following references are cited above:
[1] Rosamond, W., Flegal, K., Furie, K., Go, A., Greenlund, K., Haase, N., Hailpern, S. M., Ho, M., Howard, V., Kissela, B., Kittner, S., Lloyd-Jones, D., McDermott, M., Meigs, J., Moy, C., Nichol, G., O'Donnell, C., Roger, V., Sorlie, P., Steinberger, J., Thom, T., Wilson, M. & Hong, Y. (2008) Circulation 117, e25-146.
[2] Lodder, J. (2000) Neurosci Res Commun 26, p. 173-179.
[3] Van Reempts, J. B., M. (2000) Neurosci Res Commun 26, 161-172.
[4] Kidwell, C. S., Liebeskind, D. S., Starkman, S. & Saver, J. L. (2001) Stroke 32, 1349-59.
[5] (2008) in U01 (RFA-NS-08-001, National Institutes of Health (NIH) and Canadian Stroke Network (CSN)), pp. 1-18.
[6] Zhang, K. & Sejnowski, T. J. (2000) Proc Natl Acad Sci USA 97, 5621-6.
[7] Traystman, R. J. (2003) Ilar J 44, 85-95.
[8] Raymond, J., Darsaut, T., Salazkin, I., Gevry, G. & Bouzeghrane, F. (2008) AJNR Am J Neuroradiol.
[9] Quigley, M. (2007) J Med Ethics 33, 655-8.
[10] Bhogal, N., Hudson, M., Balls, M. & Combes, R. D. (2005) Altern Lab Anim 33, 519-27.
[11] Connors, J. J. & Wojak, J. C. (1999) Interventional neuroradiology: strategies and practical techniques (Saunders, Philadelphia).
[12] Loubinoux, I., Volk, A., Borredon, J., Guirimand, S., Tiffon, B., Seylaz, J. & Meric, P. (1997) Stroke 28, 419-26; discussion 426-7.
[13] Koizumi J, Y. Y., Nakazawa T, Ooneda G. (1986) Jpn J Stroke 8, 1-8.
[14] Longa, E. Z., Weinstein, P. R., Carlson, S. & Cummins, R. (1989) Stroke 20, 84-91.
[15] Schmid-Elsaesser, R., Zausinger, S., Hungerhuber, E., Baethmann, A. & Reulen, H. J. (1998) Stroke 29, 2162-70.
[16] Busch, E., Kruger, K. & Hossmann, K. A. (1997) Brain Res 778, 16-24.
[17] Gerriets, T., Li, F., Silva, M. D., Meng, X., Brevard, M., Sotak, C. H. & Fisher, M. (2003) J Neurosci Methods 122, 201-11.
[18] Umemura, K. & Nakashima, M. (1993) Yakubutsu Seishin Kodo 13, 9-17.
[19] Belayev, L., Alonso, O. F., Busto, R., Zhao, W. & Ginsberg, M. D. (1996) Stroke 27, 1616-22; discussion 1623.
[20] Garcia, J. H., Wagner, S., Liu, K. F. & Hu, X. J. (1995) Stroke 26, 627-34; discussion 635.
[21] Kawamura, S., Li, Y., Shirasawa, M., Yasui, N. & Fukasawa, H. (1994) Surg Neurol 41, 368-73.
[22] Kawamura, S., Yasui, N., Shirasawa, M. & Fukasawa, H. (1991) Acta Neurochir (Wien) 109, 126-32.
[23] Memezawa, H., Smith, M. L. & Siesjo, B. K. (1992) Stroke 23, 552-9.
[24] Gerriets, T., Stolz, E., Walberer, M., Muller, C., Rottger, C., Kluge, A., Kaps, M., Fisher, M. & Bachmann, G. (2004) Stroke 35, 2372-7.
[25] Freret, T., Bouet, V., Toutain, J., Saulnier, R., Pro-Sistiaga, P., Bihel, E., Mackenzie, E. T., Roussel, S., Schumann-Bard, P. & Touzani, O. (2007) J Cereb Blood Flow Metab. 26. Gao, H., Liu, Y., Lu, S., Xiang, B. & Wang, C. (2006) J Stroke Cerebrovasc Dis 15, 202-8.
[27] Liu, Y., D'Arceuil, H. E., Westmoreland, S., He, J., Duggan, M., Gonzalez, R. G., Pryor, J. & de Crespigny, A. J. (2007) Stroke 38, 138-45.
[28] Crowell, R. M., Olsson, Y., Klatzo, I. & Ommaya, A. (1970) Stroke 1, 439-48.
[29] Frazee, J. G., Luo, X., Luan, G., Hinton, D. S., Hovda, D. A., Shiroishi, M. S. & Barcliff, L. T. (1998) Stroke 29, 1912-6.
[30] Huang, J., Mocco, J., Choudhri, T. F., Poisik, A., Popilskis, S. J., Emerson, R., DelaPaz, R. L., Khandji, A. G., Pinsky, D. J. & Connolly, E. S., Jr. (2000) Stroke 31, 3054-63.
[31] Young, A. R., Touzani, O., Derlon, J. M., Sette, G., MacKenzie, E. T. & Baron, J. C. (1997) Stroke 28, 632-7; discussion 637-8.
[32] Ducruet, A. F., Mocco, J., Mack, W. J., Coon, A. L., Marsh, H. C., Pinsky, D. J., Hickman, Z. L., Kim, G. H. & Connolly, E. S., Jr. (2007) J Med Primatol 36, 375-80.
[33] Goettler, C. E. & Tucci, K. A. (2007) J Trauma 62, 777-8.
[34] Jiang, J. Y., Xu, W., Li, W. P., Xu, W. H., Zhang, J., Bao, Y. H., Ying, Y. H. & Luo, Q. Z. (2005) J Neurotrauma 22, 623-8.
[35] Bloomsmith, M. A., Brent, L. Y. & Schapiro, S. J. (1991) Lab Anim Sci 41, 372-7.
[36] Storey, P. L., Turner, P. V. & Tremblay, J. L. (2000) Contemp Top Lab Anim Sci 39, 14-6.
[37] Bland, J. M. & Altman, D. G. (1999) Stat Methods Med Res 8, 135-60.
[38] Schmued, L. C., Albertson, C. & Slikker, W., Jr. (1997) Brain Res 751, 37-46.
[39] Khanna, S., Roy, S., Slivka, A., Craft, T. K., Chaki, S., Rink, C., Notestine, M. A., DeVries, A. C., Parinandi, N. L. & Sen, C. K. (2005) Stroke 36, 2258-64.

## Claims

1. A method for testing, in a non-human mammal, an agent for treating stroke in a human, comprising the steps of:
selecting the agent and determining an experimental regimen using the agent; selecting the non-human mammal;
inducing a stroke event in the non-human animal through the substeps of:
advancing a microwire through the arterial system of the non-human mammal to a selected intracranial target position;
inserting a microcatheter along the microwire and delivering an embolic device to the target position;
occluding the artery at the target position by deploying the embolic device, verifying the occlusion and repositioning the embolic device if needed; and
withdrawing the microcatheter from the target position to an intermediate arterial location, leaving the embolic device in place; and
after a pre-determined interval, simulating reperfusion by removing the embolic device;
conducting the determined experimental regimen using the selected agent; and
assessing, after an appropriate interval, the effect of the experimental regimen on the non-human mammal, including necroptic examination.

2. The method of claim 1, wherein:
the substep of advancing the microwire comprises the steps of:
advancing a microwire through the basilar artery of the non-human mammal;
advancing the microwire from the basilar artery into and through a selected one of either the left or the right posterior communicating artery; and
advancing the microwire from the selected left or right posterior communicating artery into the corresponding left or right middle cerebral artery, which is the selected intercranial target position.

3. The method of claim 1 or claim 2, wherein:
the non-human mammal is a canine.

4. The method of claim 2 or claim 3, wherein:
in the stroke-inducing step, the substep of advancing the microwire through the basilar artery is preceded by the substeps of:
introducing the microwire into the femoral artery of the non-human animal;
and
guiding the microwire to the basilar artery from the femoral artery through the aorta, the selected one of the left and right subclavian arteries, the corresponding vertebral artery, the spinal ramus artery and the anterior spinal artery, which becomes the basilar artery.

5. The method of any one of the preceding claims, wherein:
In the microcatheter withdrawing step, the microcatheter is withdrawn at least to the origin of the basilar artery.

6. The method of any one of the preceding claims, wherein:
the stroke-inducing step comprises, during the occlusion by the embolic device, the substeps of:
performing at least one arteriogram to confirm occlusion of the ipsilateral middle cerebral artery and opacification of the contralateral hemisphere.

7. The method of any one of the preceding claims, wherein:
the embolic device is a platinum coil.

8. The method of any one of claims 2 through 7, wherein:
the M1 section of the selected middle cerebral artery remains occluded for about one hour.

9. A method for inducing an experimental stroke event in a non-human mammal, comprising the steps of:
advancing a microwire through the arterial system of the non-human mammal to a selected intracranial target position;
inserting a microcatheter along the microwire and delivering an embolic device to the target position;
occluding the artery at the target position by deploying the embolic device, confirming the occlusion and repositioning the embolic device if needed; and
withdrawing the microcatheter from the target position to an intermediate arterial location, leaving the embolic device in place; and
after a pre-determined interval, simulating reperfusion by removing the embolic device.

10. The method of claim 9, wherein:
the step of advancing the microwire comprises the substeps of:
advancing a microwire through the basilar artery of the non-human mammal;
advancing the microwire from the basilar artery into and through a selected one of either the left or the right posterior communicating artery; and
advancing the microwire from the selected left or right posterior communicating artery into the corresponding left or right middle cerebral artery, which is the selected intercranial target position.

11. The method of claim 9 or claim 10, wherein:
the non-human mammal is a canine.

12. The method of claim 10 or claim 11, wherein:
in the stroke-inducing step, the substep of advancing the microwire through the basilar artery is preceded by the substeps of:
introducing the microwire into the femoral artery of the non-human animal;
and
guiding the microwire to the basilar artery from the femoral artery through the aorta, the selected one of the left and right subclavian arteries, the corresponding vertebral artery, the spinal ramus artery and the anterior spinal artery, which becomes the basilar artery.

13. The method of any one of claims 9 to 12, wherein:
In the microcatheter withdrawing step, the microcatheter is withdrawn at least to the origin of the basilar artery.

14. The method of any one of claims 9 to 13, wherein:
the stroke-inducing step comprises, during the occlusion by the embolic device, the substeps of:
performing at least one arteriogram to confirm occlusion of the ipsilateral middle cerebral artery and opacification of the contralateral hemisphere.

15. The method of any one of claims 9 through 14, wherein:
the embolic device is a platinum coil.
